# EUROPÄISCHE PATENTSCHRIFT

(11) **EP 1 808 132 B1**
(45) Veröffentlichungstag und Bekanntmachung des Hinweises auf die Patenterteilung: **07.01.2009**
(21) Anmeldenummer: 07006418.3
(22) Anmeldetag: 24.11.2004
(51) Int. Cl.: A61B 17/02, A61F 2/46, A61B 17/16

(54) **Instrumentarium zum Formen eines Zwischenwirbelraums**
Instrument for shaping an intervertebral space
Instrument destiné à la formation d'un espace intervertébral

(30) Priorität: 10.12.2003 US 731432; 10.12.2003 US 731431
(43) Veröffentlichungstag der Anmeldung: 18.07.2007
(62) Teilanmeldung aus: 04798069.3
(73) Patentinhaber: Cervitech, Inc., Rockaway, NJ 07866 (US)
(72) Erfinder: Link, Helmut D., 22397 Hamburg (DE); McAfee, Paul C., Baltimore, MD 21204 (US); Keller, Arnold, 23863 Kayhude (DE)
(74) Vertreter: Glawe, Delfs, Moll

(56) Entgegenhaltungen:
- WO-A-20/04089258

## Beschreibung

Beim Einsetzen einer Zwischenwirbel-Gelenkprothese zum Ersatz einer Bandscheibe arbeitet der Operateur in einem sehr unübersichtlichen Operationsfeld in unmittelbarer Nähe bedeutender Nerven- und Blutbahnen. Das gilt insbesondere für den Bereich der Halswirbelsäule, weil dort die Wirbelabmessungen sehr klein und die Abstände zu sensiblen Nachbarbereichen besonders gering sind. Man ist deshalb bestrebt, die Bewegungsfreiheit von Instrumenten, die ein besonderes Gefährdungspotential haben oder die mit besonderer Genauigkeit eingesetzt werden müssen, durch geeignete Instrumente auf den notwendigen Rahmen einzuschränken. Das gilt insbesondere für Abtragwerkzeuge, die für die Formzubereitung des Wirbelzwischenraums verwendet werden. So ist es bekannt (US 20020058944 A1), für den Einsatz eines Fräsinstruments im Wirbelzwischenraum eine Führung vorzusehen, die fest mit den beteiligten Wirbelkörpern verbunden wird und ein Abweichen des Fräsinstruments von der vorgesehenen Bahn verhindert. Dieses hat ebene Ober- und Unterflächen und weist am Vorderende wenigstens zwei angetriebene Fräser auf, die scharfe Schneidkanten bilden, die einen "Vorwärtsschnitt" ermöglichen, d.h. einen das Profil des Fräsers bei dessen Vorschub abbildenden Schnitt. Dieses Profil entspricht einschließlich seiner Höhe demjenigen der vorgesehenen Prothese. Es kann eine Folge von Abtragwerkzeugen vorgesehen sein, deren Breite und/oder Dicke schrittweise zunimmt. Nachteilig ist an diesem Instrumentarium, daß die Führung den Blick auf das Operationsfeld verdeckt und daß ein Positionierfehler beim Anbringen der Führung sich auf den Schnitt des Fräswerkzeugs überträgt. Jedoch kann auf sie nicht verzichtet werden, weil die ungeführte Verwendung eines motorisch angetriebenen Fräsers zu gefährlich und fehleranfällig wäre. Bekannt ist es ferner (WO 01/62166), die Oberfläche der beteiligten Wirbelkörper mittels eines Meißels abzutragen, der gleichfalls von einer an den beteiligten Wirbelkörpern befestigten Führung geführt ist und daher auch die oben angegebenen Nachteile hat.

Der Erfindung liegt die Aufgabe zugrunde, ein Instrumentarium zum Bearbeiten der Halswirbel für das Einsetzen einer Zwischenwirbel-Gelenkprothese zu schaffen, das hohe Genauigkeit und weitestgehende Sichtkontrolle ermöglicht.

Die erfindungsgemäße Lösung liegt in den Merkmalen des Anspruchs 1. Die Genauigkeit, mit der die Prothese eingesetzt werden kann, hängt von der genauen Positionierung im Zwischenwirbelraum ab. Es ist daher wichtig, daß der Zwischenwirbelraum eine Gestalt hat, die zu der später einzusetzenden Prothese paßt. Diese wird dadurch erreicht, daß die Abtragwerkzeuge nicht von motorisch angetriebenen Fräsern, sondern durch einen Satz von mittels eines Griffs, d.h. manuell zu betätigenden Raspeln gebildet sind, deren größte jeweils im wesentlichen die Form der zu verwendenden Prothese hat, während mindestens eine weitere Raspel, die vorbereitend eingesetzt wird, etwas kleiner ist. Wenn mehrere kleinere Raspeln vorgesehen sind, werden deren Größen gegeneinander abgestuft. Die notwendige Führung wird von der knöcherne Begrenzung des Zwischenwirbelraums selbst gebildet. Dadurch kann auf eine sichtbehindernde separate Führung verzichtet werden. Auch ist die Führung durch den Knochen genauer als eine zuvor an den Wirbelkörpern anzubringende Führung. Sie wird schon bei der kleinsten Raspel wirksam, die als erste einzusetzen ist, weil schon diese den Wirbelkörpern ebene und ungezahnte Flächen zuwendet und eine Dicke hat, die derjenigen der einzusetzenden Prothese gleicht. So könnte man mit einem motorisch angetriebenen Fräser nicht verfahren, weil dieser wegen seines aggressiveren Schnitts nicht vom Knochen geführt werden kann.

Die Erfindung wird im folgenden näher unter Bezugnahme auf die Zeichnung erläutert. Darin zeigen
- Fig. 1 bis 6: einen Satz von drei unterschiedliche Raspeln,
- Fig. 7: den Umriß der Raspeln im Vergleich und
- Fig. 8 und 9: eine Prothese, für die die Raspeln bestimmt sind.

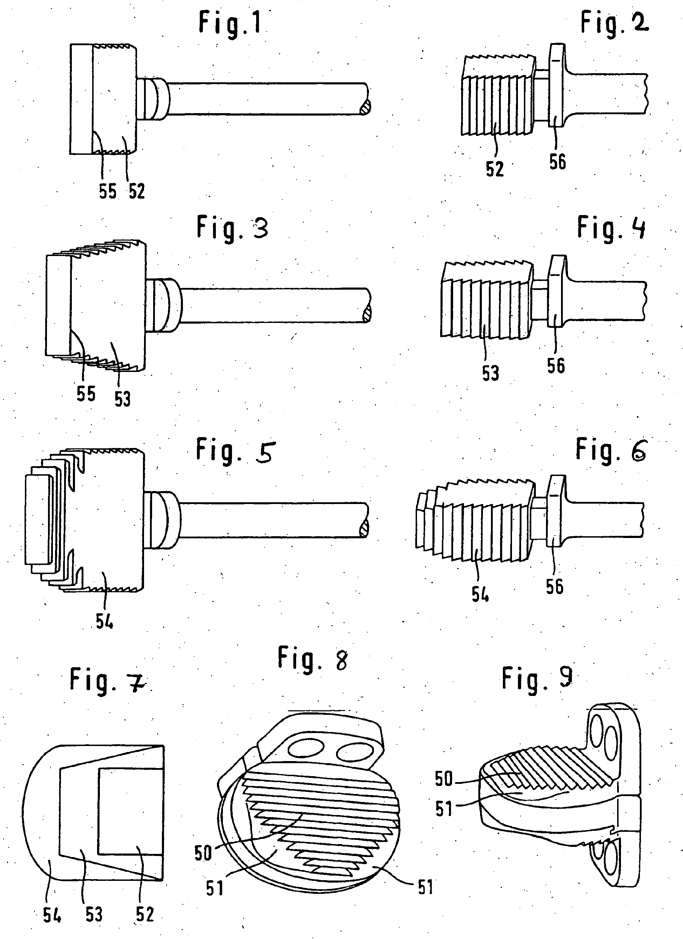

Die Wirbel, zwischen denen die in Fig.8 und 9 gezeigte Prothese eingesetzt werde soll, können mit Hilfe eines geeigneten Instruments - falls erforderlich - distrahiert werden. Ein geeignetes Instrument ist in der Veröffentlichung WO 2005/055835 gezeigt. Der zwischenwirbelraum kann in diesem zustand, in welchem die Wirbel durch das Instrument gehalten sind, zur Vorbereitung der Aufnahme der Zwischenwirbelprothese bearbeitet werden. Schließlich wird die Prothese in den Zwischenwirbelraum eingesetzt und erhält ihre endgültige Position dadurch, daß die Distraktion der Wirbel mit dem Instrument rückgängig gemacht wird.

Der Satz von Raspeln, die die Oberflächenform der Wirbel zur Aufnahme der Prothese vorbereiten, sind auf das Ausführungsbeispiel der Prothese abgestimmt, die in Fig. 8 und 9 dargestellt ist. Sie hat einen ovalen bis rechteckigen Umriß, der zur weitgehenden Ausnutzung der Ausdehnung des Zwischenwirbelraums bestimmt ist. Sie ist so flach, daß sie ohne tiefgreifende Abfräsung der Wirbelkörperdeckplatten eingesetzt werden kann. Sie wendet den Wirbelkörperdeckplatten Außenflächen zu, die in ihrem größten Teil 50 etwa eben und gezahnt sind. Ihre dorsolateralen Ecken 51 sind derart abgeschrägt, daß die Oberfläche in diesen Bereichen gegenüber der Ebene des Flächenanteils 50 zurücktritt.

Eine komplementäre Form des Zwischenwirbelraums wird durch den Satz von Raspeln 52, 53 und 54 vorbereitet, die in den Figuren 1 bis 6 dargestellt sind. Die abgestuften Größenverhältnisse der Raspeln zeigt Fig. 7. Zuerst wird die kleinste Raspel 52 mittels eines nicht detailliert dargestellten Griffs in den Zwischenwirbelraum eingestoßen, um den Zugang zu eröffnen. Es folgt Raspel 53, die Trapezform aufweist, etwa entsprechend der Trapezform des ebenen Flächenanteils der Prothesenoberfläche. Schließlich formt Raspel 54 den Zwischenwirbelraum im wesentlichen übereinstimmend mit der Form der einzusetzenden Prothese. Die Höhe aller Raspeln gleicht derjenigen der Prothese. Alle Raspeln sind in denjenigen Flächen, die dem ebenen Anteil 50 der Prothese entsprechen, ungezahnt ausgebildet. Das bedeutet, daß sie hauptsächlich mit Ihrer Vorderkante 55 eine Abrasion des Knorpels und eine Anfrischung des Knochens durchführen, ohne die Wirbelkörperendfläche wesentlich abzutragen. Alle Raspeln sind mit einem Anschlag 56 versehen, der dafür sorgt, daß sie nur bis zur vorgesehenen Tiefe in den Zwischenwirbelraum eindringen können.

## Patentansprüche

1. Instrumentarium zum Formen eines Zwischenwirbelraums entsprechend der Form einer darin einzusetzenden Zwischenwirbelprothese mittels einer Folge von nacheinander einzusetzenden Abtragwerkzeugen, die schrittweise wachsende Größe aufweisen und deren größtes jedem der beteiligten Wirbelkörper eine ebene, ungezahnte Fläche zuwendet, wobei der Höhenabstand dieser Flächen der Höhe der einzusetzenden Prothese gleicht, **dadurch gekennzeichnet, daas**
a) dass die Abtragswerkzeuge von mittels eines Griffs zu betätigenden Raspeln gebildet sind,
b) dass auch die kleineren Raspeln jedem der beteiligten Wirbelkörper eine ebene, ungezahnte Fläche zuwenden und
c) dass auch bei den kleineren Raspeln der Höhenabstand zwischen den ungezahnten und ebenen Teilen ihrer Außenfläche der Höhe der Prothese gleicht.

2. Instrumentarium nach Anspruch 1, **dadurch gekennzeichnet, dass** es einer Prothese zugeordnet ist, die den Wirbelkörpern Außenflächen zuwendet, die in ihrem größten Teil (50) eben und gezahnt und in ihren dorsolateralen Eckbereichen (51) derart abgeschrägt sind, dass sie dort gegenüber der Ebene des ebenen Teils (50) zurücktreten, und dass die größte Raspel so ausgebildet ist, dass sie den Zwischenwirbelraum übereinstimmend mit dieser Form der Prothese formt.

3. Instrumentarium nach Anspruch 1 oder 2, **dadurch gekennzeichnet, dass** die Vorderkante (55) ihrer ungezahnten Fläche abradierend ausgeführt ist.

4. Instrumentarium nach einem der Ansprüche 1 bis 3, **dadurch gekennzeichnet, dass** die zweitgrößte Raspel (53) Trapezform entsprechend der Form des ebenen Teils (50) der Prothesenoberfläche aufweist.

5. Instrumentarium nach einem der Ansprüche 1 bis 4, **dadurch gekennzeichnet, dass** alle Raspeln (52, 53, 54) mit einem ihre Eindringtiefe beschränkenden Anschlag (56) versehen sind.

## Claims

1. Instrument set for shaping an intervertebral space to match the shape of an intervertebral prosthesis that is to be inserted therein, by means of a series of material-removing tools that are to be used one after another and that are of successively increasing size, the largest of them having a flat, untoothed surface facing towards each of the vertebral bodies involved, the vertical distance between these surfaces being equal to the height of the prosthesis to be inserted, **characterized**
a) **in that** the material-removing tools are formed by rasps that are to be operated by way of a grip,
b) **in that** the smaller rasps too have a flat, untoothed surface facing towards each of the vertebral bodies involved, and
c) **in that**, in the smaller rasps too, the vertical distance between the untoothed and flat parts of their outer surfaces is equal to the height of the prosthesis.

2. Instrument set according to Claim 1, **characterized in that** it is assigned to a prosthesis with outer surfaces which face towards the vertebral bodies and which, over their greater area (50), are flat and toothed and, in their dorsolateral corner areas (51), are bevelled in such a way that they are set back there relative to the plane of the flat area (50), and **in that** the largest rasp is designed in such a way that it shapes the intervertebral space to match this shape of the prosthesis.

3. Instrument set according to Claim 1 or 2, **characterized in that** the front edge (55) of the untoothed surface is designed to be abrasive.

4. Instrument set according to one of Claims 1 to 3, **characterized in that** the second largest rasp (53) has a trapezoid shape matching the shape of the flat area (50) of the prosthesis surface.

5. Instrument set according to one of Claims 1 to 4, **characterized in that** all the rasps (52, 53, 54) are provided with an abutment (56) that limits their depth of penetration.

## Revendications

1. Instrument destiné à la formation d'un espace intervertébral correspondant à la forme d'une prothèse intervertébrale à insérer dans celui-ci, au moyen d'une succession d'outils de creusement à utiliser l'un après l'autre, qui présentent une taille progressivement croissante et dont le plus grand présente une face plane non dentée à chacun des corps de vertèbres concernés, dans lequel la distance en hauteur entre ces faces est égale à la hauteur de la prothèse à insérer, **caractérisé en ce que**
a) les outils de creusement sont formés par des râpes à manipuler au moyen d'une poignée,
b) les plus petites râpes présentent aussi une face plane non dentée à chacun des corps de vertèbres concernés, et
c) dans les plus petites râpes également, la distance en hauteur entre les parties planes et non dentées de leurs faces extérieures est égale à la hauteur de la prothèse.

2. Instrument selon la revendication 1, **caractérisé en ce qu'**il est associé à une prothèse qui présente aux corps de vertèbres des faces extérieures, qui sont planes et dentées dans leur plus grande partie (50) et qui sont chanfreinées dans leurs zones d'angle dorsolatérales (51) de telle manière qu'elles y soient en retrait par rapport au plan de la partie plane (50), et **en ce que** la plus grande râpe est configurée de telle manière qu'elle forme l'espace intervertébral concordant avec cette forme de la prothèse.

3. Instrument selon la revendication 1 ou 2, **caractérisé en ce que** l'arête antérieure (55) de sa face non dentée est réalisée de manière abrasive.

4. Instrument selon l'une quelconque des revendications 1 à 3, **caractérisé en ce que** la deuxième plus grande râpe (53) présente une forme trapézoïdale correspondant à la forme de la partie plane (50) de la surface de la prothèse.

5. Instrument selon l'une quelconque des revendications 1 à 4, **caractérisé en ce que** toutes les râpes (52, 53, 54) sont pourvues d'une butée (56) limitant leur profondeur de pénétration.
